# EUROPÄISCHE PATENTSCHRIFT

(11) **EP 0 805 348 B1**
(45) Veröffentlichungstag und Bekanntmachung des Hinweises auf die Patenterteilung: **25.02.2004**
(21) Anmeldenummer: 97105014.1
(22) Anmeldetag: 25.03.1997
(51) Int. Cl.: A61B 5/00

(54) **Anordnung zur Diagnose von malignem Gewebe durch Fluoreszenzbetrachtung**
Arrangement for the diagnosis of malignant tissue by way of flourescence observation
Disposition pour le diagnostic de tissu malignant au moyen de l'observation de la fluorescence

(30) Priorität: 29.03.1996 DE 19612536
(43) Veröffentlichungstag der Anmeldung: 05.11.1997
(73) Patentinhaber: Richard Wolf GmbH, 75438 Knittlingen (DE)
(72) Erfinder: Freitag, Lutz, Dr., 58675 Hemer (DE); Dankwart-Eder, Franz, 97270 Kist (DE)
(74) Vertreter: Vollmann, Heiko, Dipl.-Ing.

(56) Entgegenhaltungen:
- DE-A- 4 133 493
- GB-A- 2 254 417
- US-A- 4 556 057
- US-A- 5 419 312
- US-A- 5 507 287
- DATABASE INSPEC INSTITUTE OF ELECTRICAL ENGINEERS, STEVENAGE, GB Inspec No. 2995908, ANDERSSON P S ET AL: "Fluorescence endoscopy instrumentation for improved tissue characterization" XP002057412 & MEDICAL PHYSICS, JULY-AUG. 1987, USA, Bd. 14, Nr. 4, ISSN 0094-2405, Seiten 633-636,

## Beschreibung

Die Erfindung betrifft eine Anordnung zur Diagnose von malignem Gewebe durch Fluoreszenzbetrachtung gemäß den Merkmalen im Oberbegriff des Anspruches 1.

Bei der endoskopischen Tumordiagnose kann man zwischen direkten und indirekten Tumorzeichen unterscheiden. Ein direktes Tumorzeichen ist das Erkennen der Tumormasse selbst. Ein indirektes Tumorzeichen ist beispielsweise ein irregulärer Gefäßverlauf. Je nach Erkrankungsbild und -stadium des Gewebes werden jedoch die indirekten Tumorzeichen immer weniger spezifisch. Frühkarzinome, das heißt röntgennegative und endoskopisch nicht mehr von gesundem Gewebe unterscheidbare Gewebsveränderungen können dann nur noch schwer diagnostiziert werden. Hier nutzt man Fluoreszenzuntersuchungen, um in gesunder Umgebung veränderte Zell- oder Gewebestrukturen zu ermitteln. Dabei wertet man das unterschiedliche Fluoreszenzverhalten von gesundem und krankhaftem Gewebe aus. Neben der Messung und Auswertung der Autofluoreszenz ist es auch bekannt, den Kontrast durch Verabreichen von photosensiblen Substanzen mit starker Affinität zum malignen Gewebe zu verstärken. Man spricht dann von medikamenteninduzierter Fluoreszenz bzw. photodynamischer Diagnostik. Diese Substanzen werden im malignen Gewebe länger gespeichert als in der gesunden Umgebung. Durch Lichtexposition im ultravioletten bis roten Bereich, welches üblicherweise durch einen Laser erzeugt wird, wird die Fluoreszenz stimuliert. Eine Analyse der Wellenlänge der Fluoreszenz, die vom geschädigten Gewebe ausgeht, ermöglicht dann die Diagnose von Tumoren. Die Autofluoreszenz und die medikamenteninduzierte Fluoreszenz ermöglicht somit weitere Informationen, die mit dem bloßen Auge nicht erfaßt werden können.

Die US-PS 4 556 057 offenbart die Mittel zur Durchführung einer endoskopischen Tumordiagnose einschließlich der Mittel zum Anregen der Fluoreszenz, zur Spektralanalyse und die erforderlichen Lasergeräte. Hierbei wird ein Endoskop mit einer optischen Faser in eine Körperhöhle eingeführt und das zu untersuchende Gewebe mit einem pulsierenden Laserlicht bestrahlt. Das induzierte Fluoreszenzlicht wird dann gemessen und ausgewertet, um zu bestimmen, ob ein Gewebe normal oder krankhaft ist. Erkranktes Gewebe kann anschließend durch Bestrahlen mit hoher Laserenergie durch dieselbe optische Faser zerstört werden.

Die Untersuchung wird in dem monochromatischen Lichtspektrum durchgeführt, bei dem sich eine Fluoreszenz anregen läßt. Von dem erkrankten Gewebe kann ein Bild aufgezeichnet werden. Hierfür ist ein Bildverstärker notwendig, weil das interessierende Fluoreszenzsignal mehrere Zehnerpotenzen niedriger ist als das, was mit dem bloßen Auge erkannt werden kann.

Eine zeitgleiche Betrachtung mit weißem Licht kann nicht vorgenommen werden. Um ein aufgefundenes möglicherweise krankhaftes Gewebe örtlich zu lokalisieren, ist eine Weißlichtquelle vorgesehen, die alternierend mit dem gepulsten Laserstrahl ansteuerbar ist.

Eine gleichzeitige Auswertung der optisch indirekten Tumorzeichen und der Fluoreszenztumorzeichen ist bislang nicht möglich. Dies wäre jedoch wünschenswert, da beispielsweise auch Dysplasien und Entzündungen Fluoreszenz aufweisen. Desweiteren würde der gesamte Diagnosevorgang und das Abtasten in der Körperhöhle erleichtert bzw. verbessert.

Die bekannte Anordnung ist auch nicht zur Tumoruntersuchung bei Autofluoreszenz geeignet, weil man die Energie des zur Fluoreszenzanregung genutzten Lichts nicht begrenzen kann. Dies ist deshalb erforderlich, weil die Zellen ihre Fluoreszenzfähigkeit verlieren, wenn ein monochromatisches Licht zur Fluoreszenzanregung von biologischem Gewebe eine bestimmte Energie bzw. Intensitätsspitze überschreitet. Diesen Verlust der Fluoreszenzfähigkeit nennt man Bleaching.

Es ist daher Aufgabe der Erfindung, eine Anordnung zu schaffen, durch die die Erkennungsmöglichkeiten von gegenüber gesundem Gewebe veränderten Zell- oder Gewebestrukturen mittels Fluoreszenzuntersuchung verbessert werden, wobei gleichzeitig mit weißem Licht ein normales optisches Bild des zu untersuchenden Gewebes betrachtet werden kann.

Die Lösung des gegenständlichen Teils dieser Aufgabe besteht in den im kennzeichnenden Teil des Anspruchs 1 aufgeführten Merkmalen.

Kernpunkt der Erfindung bildet die Maßnahme, eine Weißlicht erzeugende Lichtquelle einzusetzen, die eine Vorrichtung zur periodischen Intensitätsänderung des von der Lichtquelle ausgehenden Lichtbündels umfaßt, während vom Fluoreszenzstimulator ständig ein kontinuierliches Lichtbündel ausgeht. Die Intensitätsänderung kann sowohl durch eine Unterbrechung des Lichtbündels als auch durch eine steuertechnische Regelung des Lichtbündels bzw. der Weißlichtquelle bewirkt werden.

Die Erfindung macht sich dabei den an sich bekannten physikalischen Effekt zu eigen, daß das menschliche Auge oberhalb der Flimmergrenze ein stillstehendes Bild zusammensetzt und daß dieser Effekt auch auf das Bildaufzeichnungsgerät bzw. einen Videochip anwendbar ist. Hierdurch ergibt sich erfindungsgemäß der Vorteil, daß die Fluoreszenz, die man analysieren will, auch wenn sie um Zehnerpotenzen unter der sichtbaren Lichtwellenlänge liegt, betrachtet werden kann. Die Sichtbarkeitsschwelle wird so nach unten verlagert.

Das weiße Licht kann grundsätzlich von einer weißen Blitzröhre erzeugt werden. Es kann aber auch durch die additive Mischung von drei Spektralfarben Rot, Grün und Blau generiert werden.

Vom Fluoreszenzstimulator geht während der Diagnose ständig ein kontinuierliches Lichtbündel aus mit einer Wellenlänge, die die Fluoreszenz des zu untersuchenden Gewebes anregt. Vorzugsweise handelt es sich dabei um monochromatisches Licht. Grundsätzlich ist aber auch die Einspeisung von polychromatischem Licht möglich.

Durch die Intensitätsänderung des von der Weißlichtquelle ausgehenden Lichtbündels wird im Zyklus eine Dunkelphase gewonnen, die zur Analyse der vom Gewebe ausgehenden Wellenlänge der Fluoreszenz genutzt werden kann. Als Analysator kommt üblicherweise ein Spektrometer zum Einsatz. Grundsätzlich kann aber auch eine Kamera für ein Vollbild eingesetzt werden.

Der Analysator ist logisch so in die erfindungsgemäße Anordnung eingebunden, daß er bei gegen Null gehender Intensität des Lichtbündels der Weißlichtquelle aktiviert und bei einem sichtbaren Lichtpuls deaktiviert ist. Hierdurch wird vermieden, daß der Analysator von einem Lichtpuls überstrahlt wird, wodurch er für eine gewisse Zeit für die Analyse nicht brauchbar wäre. Unter Aktivierung bzw. Deaktivierung des Analysators ist eine Steuerung der Empfindlichkeit des Analysators zu verstehen. Der Analysator muß also in einer deaktiven Phase nicht aus sein, sondern in seiner Empfindlichkeit lediglich so weit herabgesetzt sein, daß seine Arbeitsfähigkeit durch den Lichtimpuls nicht beeinträchtigt wird. Dies kann auch durch Vorschaltung geeigneter Filter erfolgen.

Erfindungsgemäß wird somit eine kontinuierliche Lichtquelle zur Fluoreszenzstimulation und eine sehr starke, ultrakurze Weißlichtquelle kombiniert, wobei die Fluoreszenzanalyse in der Dunkelphase zwischen den Weißlichtimpulsen stattfindet.

Nach den Merkmalen des Anspruchs 2 ist die Lichtquelle Bestandteil eines Stroboskops. Das Stroboskop wird auf das Beleuchtungsbündel aufgekoppelt und ersetzt das bislang verwendete Dauerlicht. Durch die Lichtpulsfolge mit hoher Intensität extrem kurzer Dauer wird ein Weißlichtbild geschaffen, mit dem die normale anatomisch-physiologisch leicht gerötete Schleimhaut betrachtet werden kann.

Das zu untersuchende Gewebe wird von der Stroboskoplampe im Takt der Steuerfrequenz angeleuchtet. Die Steuerfrequenz liegt hierbei vorzugsweise höher als 15 Hz. Die Dauer eines Lichtblitzes kann je nach Blitzröhre zwischen 1 µs und 100 µs liegen.

Der Stroboskopeffekt, der auch das normale menschliche Auge täuscht, ist ebenfalls anwendbar auf einen Videochip bzw. eine Videoröhre. Die Lichtpulse auf den Videochip bzw. die Videoröhre sind von extrem kurzer Dauer. Zwischen den Lichtpulsen des Weißlichts herrscht Dunkelheit. Während dieser Dunkelphase kann die Fluoreszenz analysiert werden, da der Fluoreszenzstimulator dauernd anliegt. Für den Betrachter ergibt sich jedoch während der gesamten Zykluszeit ein Bild mit pseudoweißem Licht. Bei einem Verhältnis der Belichtungsphase zur Dunkelphase von beispielsweise 1:1000 herrscht somit in 99,9 % der Gesamtphase Dunkelheit, die als Fluoreszenzzeit zur Analyse genutzt werden kann. Das Empfindlichkeitsdefizit bei der Fluoreszenzanalyse wird damit durch die Analysenzeit ausgeglichen.

Als Fluoreszenzstimulator kommt nach den Merkmalen des Anspruchs 3 ein Laser zum Einsatz. Hierbei kann es sich beispielsweise um einen Krypton- oder Argon-Ionenlaser handeln. Das vom Laser ausgehende Licht ist so zu wählen, daß eine gute Autofluoreszenz angeregt wird. Bei medikamenteninduzierter Fluoreszenz ist das Licht auf das jeweilige Medikament abzustimmen.

Viele Lasermaterialien können Verstärkung für eine ganze Reihe verschiedener Lichtfrequenzen liefern. Um in diesen Fällen eine monochromatische Emission auf einer gewünschten Wellenlänge zu erhalten, müssen zur Unterdrückung der anderen Wellenlängen Filter (Prismen, Gitter, Interferenzfilter und anderes) eingesetzt werden. Da bei der erfindungsgemäßen Anordnung der Laser kontinuierlich läuft, können vom Prinzip her beliebige Wellenlängen genutzt werden. Bei kontinuierlicher Variation der Durchlaßwellenlänge des eingesetzten Filters können dann breite Bereiche des Spektrums lückenlos mit monochromatischer Lichtstrahlung überdeckt werden.

Die Erfindung ermöglicht aber auch den Einsatz einer Bogenlampe, beispielsweise einer Xenon- oder Quecksilberdampflampe, als Fluoreszenzstimulator, wie es Anspruch 4 vorsieht. Damit steht eine Lichtquelle zur Verfügung mit sehr ausgeprägtem Spektrum im blauen Bereich. Diese Lichtquelle ist gegenüber einem Laser wesentlich preisgünstiger.

Eine den allgemeinen Erfindungsgedanken weiterbildende Ausführungsform ist in Anspruch 5 charakterisiert. Danach ist dem Analysator ein Filter zugeordnet, der die vom Fluoreszenzstimulator ausgehende Anregungswellenlänge selektiv ausblendet. Bei dem Filter kann es sich um Kantenfilter, Interferenzfilter, Prismen oder Gittersysteme und ähnliches handeln.

Besonders vorteilhaft wird sowohl auf der Anregungsseite, also in das vom Fluoreszenzstimulator ausgehende Lichtbündel, als auch auf der Analyseseite ein System von wenigstens zwei Filtern eingegliedert, welche mechanisch oder elektronisch so verkoppelt sind, daß die vom Gewebe ausgehende Wellenlänge der Fluoreszenz, welche der Anregungswellenlänge entspricht, vor dem Analysator selektiv ausgeblendet wird. Bei Einsatz eines Array-Spektrometers (OMA = Optical Multichannel Analyzer) oder auch eines Interferenzspektrometers kann somit das gesamte Spektrum simultan analysiert werden.

Möglich ist es auch, daß ein Spektrometersensor auf der Analyseseite direkt mit dem Filter auf der Anregungsseite verknüpft wird. Durch die Kopplung der Filter auf der Anregungs- und auf der Analyseseite können nunmehr beliebige Lichtquellen für die Diagnose verwendet werden. Somit können auch vergleichsweise preisgünstige Lichtquellen zum Einsatz gelangen.

Bei dem entsprechenden Verfahren zur Diagnose von malignem Gewebe durch Fluoreszenzbetrachtung wird ein Stroboskop benutzt, um die interessierende Region in einer Körperhöhle durch ein Endoskop zu betrachten. Die Blitzfolgefrequenz liegt vorzugsweise über 15 Hz. Gleichzeitig wird eine monochromatische Lichtquelle, beispielsweise ein Argonlaser, benutzt, um die Autofluoreszenz kontinuierlich zu stimulieren. Das Weißlichts und das Laserlicht können grundsätzlich über ein und dieselbe Lichtleitfaser im Endoskop eingekoppelt werden. Selbstverständlich ist es auch möglich, separate Lichtleitfasern zu verwenden.

Durch die Folge von Lichtblitzen hoher Intensität extrem kurzer Dauer wird ein Weißlichtbild geschaffen. Zwischen den einzelnen Blitzen ergibt sich eine vergleichsweise lange Lesezeit für den Videochip (CCD-Bildwandlerchip) bzw. die Videoröhre. Die Intensitätsdifferenz zwischen den vom Weißlicht ausgehenden Signalen und den vom Fluoreszenzlicht ausgehenden Signalen wird überwunden durch die Unterschiede in der Betrachtungs- und der Analysezeit. Das aufgenommene Spektrogramm kann dann auf einem Bildschirm angezeigt werden, der ein normales Weißlichtendoskopbild wiedergibt.

Durch den Einsatz von Filtern, die logisch dergestalt verknüpft sind, daß vor dem Analysator jeweils die vom Gewebe ausgehende Wellenlänge ausgeblendet wird, welche der Anregungswellenlänge entspricht, ist es möglich, bei kontinuierlicher Variation der Durchlaßwellenlänge breite Bereiche des Spektrums lückenlos mit monochromatischer Lichtstrahlung zu überdecken. Damit ist ein sogenanntes Spektrumscanning möglich. Die Anregung kann dann mit Wellenlängen in unterschiedlichen Spektralbereichen, beispielsweise blau, gelb, rot etc., erfolgen.

Elektronische oder mechanische Rot-Grün-Blau-Filter sind für den Einsatz bei der erfindungsgemäßen Anordnung sehr gut geeignet.

Insgesamt kann durch die erfindungsgemäße Anordnung die benötigte Untersuchungszeit wesentlich verkürzt werden. Da ein Realbild betrachtet werden kann, ist die Untersuchung erheblich einfacher und zuverlässiger.

Grundsätzlich ist es erfindungsgemäß auch möglich, statt der bisher beschriebenen Reflexion des Gewebes die Absorption des Gewebes zu messen. Hierfür wird in das Gewebe eingestochen. Dies kann einmal dadurch erfolgen, daß die Lichtleitfaser in das Gewebe eingestochen wird, über die der Laserstrahl geleitet wird. In diesem Fall würde das gesamte Gewebe leuchten. Durch die unterschiedliche Absorption von gesundem und krankhaftem Gewebe lassen sich beispielsweise Tumorränder klarer erkennen. Weiterhin ist es auch möglich, die Analysefaser einzustechen, um die Absorption direkt zu messen.

Die erfindungsgemäße Anordnung kann geeigneterweise durch Mittel zum Bestrahlen mit hoher Energie durch dieselbe Lichtleitfaser ergänzt werden. Damit kann nach der Diagnose unmittelbar eine Zerstörung und Entfernung des erkrankten Gewebes vorgenommen werden. Hierbei kann derselbe Laser sowohl für die Diagnosezwecke als auch für die Bestrahlung verwendet werden, wenn die Laserenergie entsprechend einstellbar ist.

Die Erfindung ist nachfolgend anhand eines Ausführungsbeispiels beschrieben. Es zeigen:
- Figur 1: in technisch generalisierter Darstellungsweise eine Anordnung zur Diagnose von malignem Gewebe durch Fluoreszenzbetrachtung;
- Figur 2: in schematischer Darstellung die während eines Zeitraums ablaufenden Aktiv- und Passivphasen von Laser, Stroboskop, Spektrometer und Videochip;
- Figur 3: eine weitere Ausführungsform einer erfindungsgemäßen Anordnung;
- Figur 4: ein erstes Filterrad der Anordnung gemäß Figur 3;
- Figur 5: ein zweites Filterrad der Anordnung gemäß Figur 3;
- Figur 6: ein Impulsdiagramm;
- Figur 7: eine weitere Ausführungsform eines Filterrads und
- Figur 8: ebenfalls in technisch generalisierter Darstellungsweise eine weitere Anordnung zur Diagnose von malignem Gewebe durch Fluoreszenzbetrachtung.

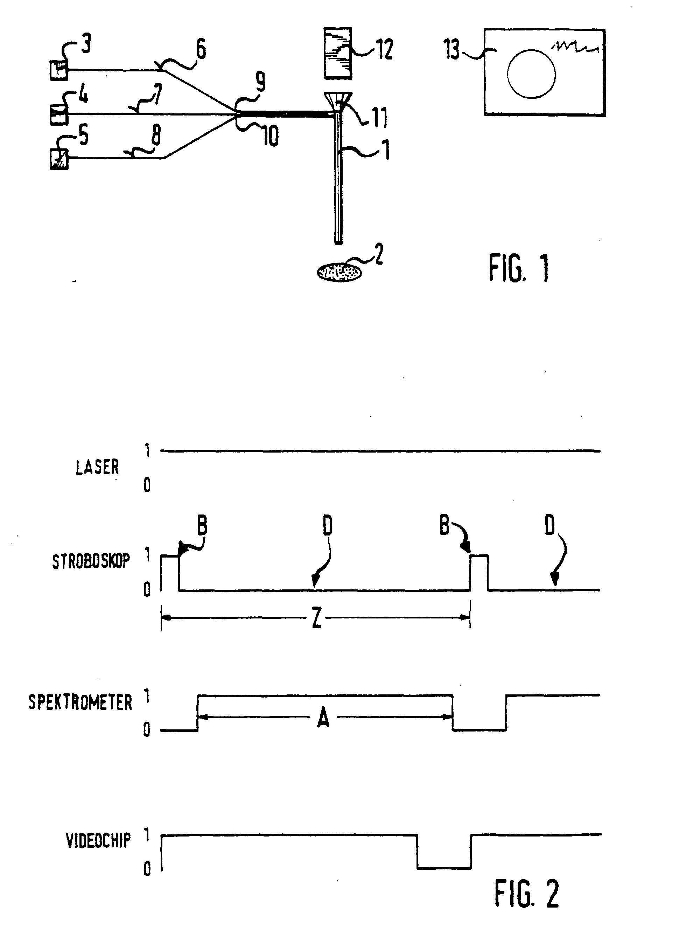

In der Figur 1 ist mit 1 ein flexibles oder starres Endoskop bezeichnet, welches in eine nicht näher dargestellte Körperhöhle zur Untersuchung des Gewebes 2 eingeführt ist.

Weiterhin ist eine Weißlichtquelle in Form eines Stroboskops 3, ein die Fluoreszenz des betrachteten Gewebes 2 stimulierender Laser 4 und ein Spektrometer 5 dargestellt. Die Anbindung von Stroboskop 3, Laser 4 und Spektrometer 5 ist technisch vereinfacht durch die Linien 6, 7 und 8 veranschaulicht.

Grundsätzlich reicht für die Lichtleitung vom Stroboskop und Laser bzw. zum Spektrometer eine einzige Lichtleitfaser aus. Eine zweckmäßige Lösung besteht jedoch darin, das Licht von Stroboskop 3 und Laser 4 über eine gemeinsame Lichtleitfaser 9 zu führen und für die Analyse eine separate Analysefaser 10 vorzusehen.

Vom Laser 4 geht während der Diagnose ständig ein kontinuierlicher Laserstrahl aus, um die Autofluoreszenz des Gewebes 2 zu stimulieren. Gleichzeitig wird das Stroboskop 3 benutzt, um das interessierende Gewebe 2 durch das Endoskop 1 zu beleuchten. Durch entsprechende Wahl der Taktfrequenz des Stroboskops 3 wird so ein Weißlichtbild geschaffen, welches dem normalen anatomisch-physiologischen Echtbild entspricht. Die Analyse der vom Gewebe 2 ausgehenden Wellenlänge der Fluoreszenz wird vom Spektrometer 5 durchgeführt. Hierzu sind das Stroboskop 3 und der Spektrometer 5 logisch so verknüpft und ansteuerbar, daß der Spektrometer jeweils in einer Dunkelphase aktiviert und bei einem Lichtblitz deaktiviert ist.

Das Endoskop 1 verfügt über einen Bildwandler, der eine Bildschirmwiedergabe ermöglicht. Dieser ermöglicht eine Zerlegung des vom Objektiv entworfenen Bilds in einzelne Bildelemente und ihre Umwandlung in elektrische Signale. Die elektrischen Signale werden in integrierten ladungs-gekoppelten Bauelementen vorübergehend gespeichert, ehe sie durch hochfrequente Taktimpulse abgerufen und in Vi-deosignale umgewandelt werden.

Während der Diagnose kann über das Okular ii das zu untersuchende Gewebe betrachtet werden, und zwar als Echt-bild entweder mit dem Auge oder über eine Endokamera 12. Auf einem Bildschirm 13, der ein normales Weißlichtendo-skopbild wiedergibt, kann das aufgenommene Spektrogramm angezeigt werden.

Das Signal, das nach der Auswertung und Aufbereitung durch den Spektrometer 5 vorliegt, kann in das Untersu-chungsbild eingeblendet werden. Es kann aber auch in Form eines akustischen Signals ausgegeben werden. Der Untersu-cher erkennt dann anhand der Signaländerung, daß bei dem untersuchten Gewebe eine Anomalie vorliegt. In einer weiteren Ausgestaltung ist vorgesehen, daß durch permanente Aufzeichnung des Spektrums ein Gewebemuster ermittelt wird, welches dem Normalspektrum des jeweiligen Patienten entspricht. Ändert sich dann im Verlauf der Diagnose dieses individuelle Muster, wird ein entsprechendes Signal ausgegeben. Die erfindungsgemäße Anordnung verfügt dann über einen Selbstlernalgorhitmus.

In der Figur 2 sind die Aktivphasen der Fluoreszenzstimulation durch den Laser, des Stroboskops, der Fluoreszenzanalyse durch das Spektrometer und des Videochips jeweils durch die Stellung (I) und deren Passivphasen durch die Stellung (O) gekennzeichnet.

Hieraus wird deutlich, daß der Laser während der Diagnose kontinuierlich läuft, um die Autofluoreszenz des Gewebes zu stimulieren. Das Stroboskop wird benutzt, um die interessierende Gewebsregion durch das Endoskop zu beleuchten. Jeweils in der Dunkelphase D zwischen zwei Blitzen B ist das Spektrometer aktiv. Die Dunkelphase D steht als Analysezeit A zur Verfügung, weil der Laser dauernd anliegt. Der Videochip ist jeweils zu Beginn eines Blitzes B aktiv und schaltet sich kurz vor dem Ende der Analysezeit ab. Die elektronischen Schaltzeiten sind bei diesen Vorgängen entsprechend berücksichtigt.

Bei einer Blitzdauer von beispielsweise 1 Promille eines Zyklusses Z stehen somit 999 Promille der Zykluszeit zur Analyse zur Verfügung. Die Intensitätsdifferenz zwischen den Weißlichtsignalen und den Fluoreszenzsignalen kann demgemäß durch den Unterschied in der Betrachtungszeit und der Analysezeit überwunden werden.

Für den Betrachter ergibt sich somit während der gesamten Zykluszeit ein Bild mit pseudoweißem Licht. Dieses kann auf einen Bildschirm übertragen werden, der ein normales Weißlichtendoskopbild zeigt. Damit ist eine Fluoreszenzdiagnose unter Weißlicht möglich, so daß während der Diagnose die gleichzeitige Information der optisch indirekten Tumorzeichen und der Fluoreszenztumorzeichen ausgewertet werden können. Diese synergistische Kombination steigert die Diagnosemöglichkeiten und macht es möglich, unterschiedliche Krebsphänomene zu erkennen.

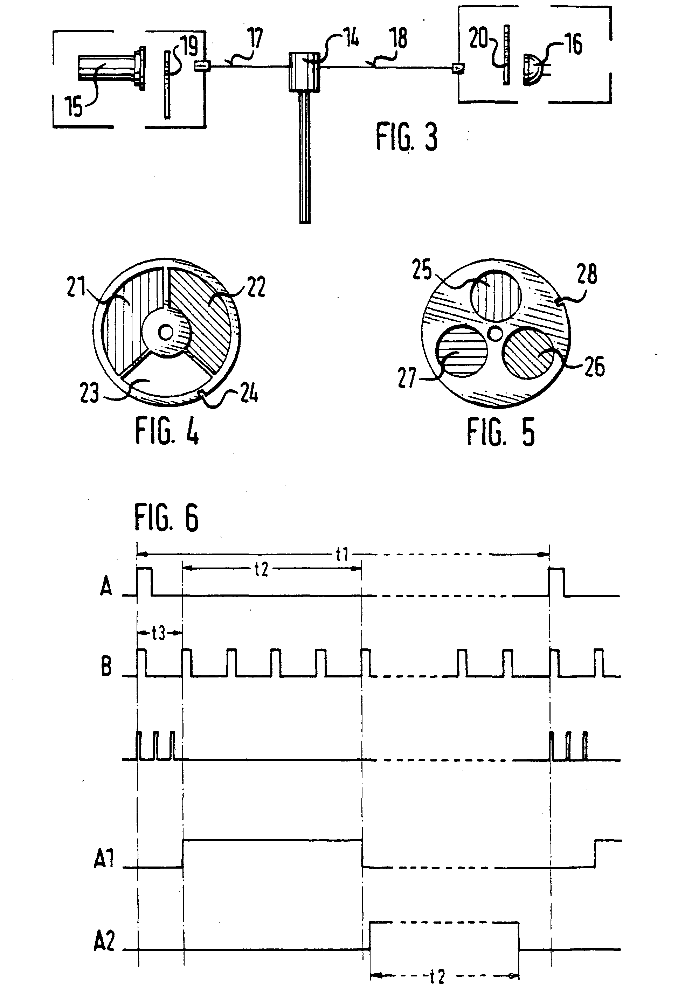

Die Figur 3 zeigt eine Anordnung zur Diagnose von malignem Gewebe durch Fluoreszenzbetrachtung mit einem Endoskop 14, welches mit einer Kamera 15 und einer Blitzlichtquelle 16 mittels der Lichtleiter 17 bzw. 18 gekoppelt ist. <

Der Kamera 15 ist ein Filtersystem 19 in Form des Filterrads A zugeordnet, wohingegen die Blitzlichtquelle 16 mit einem Filtersystem 20 (Filterrad B) zusammenarbeitet.

Filterrad A und Filterrad B sind in den Figuren 4 und 5 erläutert.

Das Filterrad A enthält einen ersten Filter 21, z.B. für rotes Licht (Filter A1), und einen zweiten Filter 22, z.B. für grünes Licht (Filter A2). Weiterhin ist eine Öffnung 23 und eine Kodierkerbe 24 auf dem Außenumfang des Filterrads A vorhanden. Die Kodierkerbe 24 dient der Synchronisation vom Filterrad A mit dem Filterrad B. In die Öffnung 23 kann ein Graufilter zur Intensitätsanpassung eingebaut sein.

Das Filterrad B ist mit drei Rundfiltern 25, 26, 27 ausgerüstet für die Spektralfarben Rot (R), Grün (G), Blau (B). Durch die Signale RGB wird ein pseudoweißes Licht zusammengesetzt. Auch das Filterrad B ist mit einer Kodierkerbe 28 versehen.

Das Zusammenwirken und die Arbeitsweise der vorstehend beschriebenen Anordnung ist anhand des Impulsdiagramms der Figur 6 verdeutlicht.

Bei einer Bildwiederholungsfrequenz eines Monitors von 50 Hz ist die Zeit t1 = 20 ms. Während dieser Zeit werden fünf Bilder von der Kamera 15 aufgenommen. Die ersten drei Bilder sind die Aufnahmen der durch die Filter 25, 26, 27 gefärbten Blitze während der Zeit t3.

Blitzlichtquelle 16, Kamera 15 sowie die Filtersysteme 19, 20 sind so synchronisiert, daß die Kamera 15 gerade dann das optische Signal verarbeitet, wenn ein Blitz ausgelöst ist und sich das jeweilige Filter 25-27 vor der Blitzlichtquelle 16 befindet. Im Strahlengang vor der Kamera 15 ist zu dieser Zeit jeweils kein Farbfilter 21 oder 22 eingeschwenkt.

Bei einer maximalen Blitzfrequenz von beispielsweise 1.000 Hz ist die Zeit t3 = 3 ms. Das Filterrad B muß sich dann mit 18.000 U/min drehen. Für die Messung der Fluoreszenz verbleiben somit 17 ms. Die Analysezeit ist hierbei unterteilt in die Sammelzeit für die rote Fluoreszenz und die Sammelzeit für die grüne Fluoreszenz. Bei Berücksichtigung der Übergangszeiten von Filter 21 zu 22 des Filterrads A verbleiben für die Zeit t2 noch jeweils etwa 5 ms bis 6 ms.

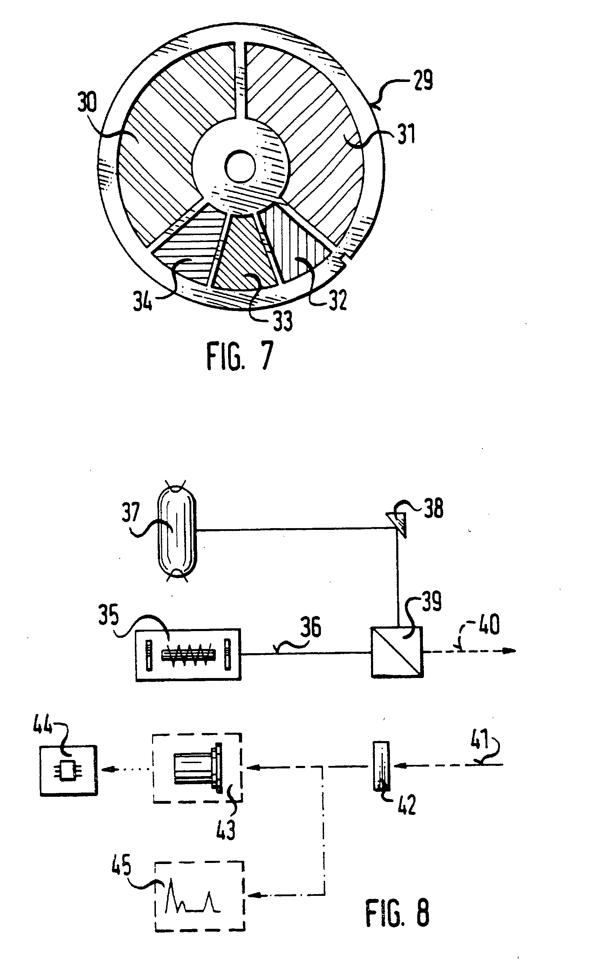

In der Figur 7 ist ein Filterrad 29 dargestellt, welches ebenfalls bei einer Anordnung gemäß der Figur 3 zum Einsatz gelangen kann. In diesem Fall ist kein Filtersystem für die Blitzlichtquelle erforderlich. Das Filterrad B entfällt dann.

Das Filterrad 29 weist ein erstes Meßfilter 30 und ein zweites Meßfilter 31 zur Fluoreszenzmessung auf. Zur Weißlichtbetrachtung sind die Filter 32, 33, 34 für die Spektralfarben Rot (R), Grün (G) und Blau (B) vorhanden.

Bei einer solchen Anordnung entspricht das Impulsdiagramm demjenigen der Figur 6, wobei die Synchronisationsimpulse von Filterrad B entfallen, da dieses nicht vorhanden ist. Die Blitzfolge der Blitzlichtquelle 16 ist dann so synchronisiert, daß die Blitze ausgelöst werden, wenn jeweils ein Filter 32, 33 oder 34 im Strahlengang der Kamera 16 ist.

Ein weiteres Ausführungsbeispiel der Erfindung ist in der Figur 8 dargestellt.

Mit 35 ist eine monochromatische Lichtquelle bezeichnet, von der ein Dauerlicht 36 im blauen Spektralbereich mit einer Wellenlänge von vorzugsweise 488 nm ausgeht. Der für eine zeitgleiche Betrachtung des zu untersuchenden Gewebes mit weißem Licht erforderliche Lichtanteil wird durch die Blitzröhre 37 erzeugt.

Mit Hilfe eines Spiegels 38 werden die Lichtblitze umgeleitet und im Bildteiler 39 mit der von der Lichtquelle 35 ausgehenden Lichtphase 36 gekoppelt. Der sich aus den Lichtanteilen zusammensetzende Strahl 40 wird dann über eine hier nicht dargestellte Lichtleitfaser mittels eines Endoskops auf das zu untersuchende Gewebe geleitet.

Vom Gewebe aus ergibt sich eine Lichtbildreflektion gemäß dem mit 41 gekennzeichneten Strahl, der einen Echtlichtanteil und einen Fluoreszenzlichtanteil aufweist. Diese Lichtanteile werden in einem RGB-Filter 42 getrennt. Hierbei ergeben sich Rot-Grün-Blau-Signale, die durch ein Bildaufnahmeelement 43 aufgenommen und durch einen diesem zugeordneten Prozessor 44 entsprechend ausgewertet werden.

Bei dem Bildaufnahmeelement 43 kann es sich beispielsweise um eine hochempfindliche Schwarz/Weiß-Kamera oder einen CCD-Chip handeln.

Grundsätzlich kann auch eine direkte Auswertung der gewonnenen Signale in einem Spektrometer 45 erfolgen.

Der RGB-Filter 42 und der Prozessor 44 sind synchronisiert, so daß jeweils entsprechend gerade der Lichtanteil, der vom Filter 42 durchgelassen wird, als entsprechendes Signal aufgezeichnet und vom Prozessor 44 im RGB-Modulationsverfahren ausgewertet wird. Folglich werden insgesamt fünf Signale genutzt, nämlich die drei RGB-Signale als simuliertes Weißlicht und der Rot- und Grünanteil aus dem Fluoreszenzlicht.

Der Videochip bleibt hier immer empfindlich. Die Lesezeit wird durch den RGB-Filter 42 reguliert, wobei die Lesezeit während der Analysephase geteilt ist in die Fluoreszenzzeit rot und die Fluoreszenzzeit grün. Der Weißlichtanteil ist gegenüber dem Fluoreszenzlichtanteil gering, damit man die Empfindlichkeitsdifferenz zur Analyse ausnutzen kann.

Der Einsatz einer Schwarz/Weiß-Kamera hat den Vorteil, daß diese gegenüber einer Farbkamera nicht nur erheblich kostengünstiger ist, sondern auch wesentlich leichter und handlicher ist. Sie kann auch mit einem Lichtverstärker ausgerüstet werden, um die Empfindlichkeit weiter zu steigern.

### Bezugszeichenaufstellung

- 1 -: Endoskop
- 2 -: Gewebe
- 3 -: Stroboskop
- 4 -: Laser
- 5 -: Spektrometer
- 6 -: Linie
- 7 -: Linie
- 8 -: Linie
- 9 -: Lichtleitfaser
- 10 -: Lichtleitfaser
- 11 -: Okkular
- 12 -: Endokamera
- 13 -: Bildschirm
- 14 -: Endoskop
- 15 -: Kamera
- 16 -: Blitzlichtquelle
- 17 -: Lichtleiter
- 18 -: Lichtleiter
- 19 -: Filtersystem
- 20 -: Filtersystem
- 21 -: Filter
- 22 -: Filter
- 23 -: Öffnung
- 24 -: Kodierkerbe
- 25 -: Filter
- 26 -: Filter
- 27 -: Filter
- 28 -: Kodierkerbe
- 29 -: Filterrad
- 30 -: Meßfilter
- 31 -: Meßfilter
- 32 -: Filter
- 33 -: Filter
- 34 -: Filter
- 35 -: monochromatische Lichtquelle
- 36 -: Dauerlicht
- 37 -: Blitzröhre
- 38 -: Spiegel
- 39 -: Bildteiler
- 40 -: Strahl
- 41 -: Strahl
- 42 -: RGB-Filter
- 43 -: Bildaufnahme
- 44 -: Prozessor
- 45 -: Spektrometer

- A -: Analysezeit
- B -: Blitz
- D -: Dunkelphase
- Z -: Zyklus
- t1 -: Zeit
- t2 -: Zeit
- t3 -: Zeit

## Patentansprüche

1. Anordnung zur Diagnose von malignem Gewebe durch Fluoreszenzbetrachtung mit einem Endoskop (1), welche eine Weißlichtquelle (3, 16, 37), einen ein Lichtbündel aussendenden Fluoreszenzstimulator (4, 35) und einen Analysator (5, 15, 43) aufweist, die Weißlichtquelle (3, 16, 37) eine Vorrichtung zur periodischen Intensitätsänderung des von der Weißlichtquelle (3, 16, 37) ausgehenden Lichtbündels umfaßt und der Analysator (5, 15, 43 bei gegen Null gehender Intensität des Lichtbündels der Weißlichtquelle (3, 16, 37) aktivierbar und bei einem sichtbaren Lichtpuls deaktivierbar ist, **dadurch gekennzeichnet, daß** vom Fluoreszenzstimulator (4, 35) während der Diagnose ständig ein kontinuierliches Lichtbündel auf das Gewebe ausgeht.

2. Anordnung nach Anspruch 1, **dadurch gekennzeichnet, daß** die Weißlichtquelle (3, 16, 37) Bestandteil eines Stroboskops ist.

3. Anordnung nach Anspruch 1 oder 2, **dadurch gekennzeichnet, daß** der Fluoreszenzstimulator (4) ein Laser ist.

4. Anordnung nach Anspruch 1 oder 2, **dadurch gekennzeichnet, daß** der Fluoreszenzstimulator eine Bogenlampe ist.

5. Anordnung nach einem der Ansprüche 1 bis 4, **dadurch gekennzeichnet, daß** dem Analysator (5, 15, 43) ein Filter (19, 42) zugeordnet ist, der die vom Fluoreszenzstimulator (4, 35) ausgehende Anregungswellenlänge selektiv ausblendet.

## Claims

1. An arrangement for the diagnosis of malignant tissue by way of fluorescence observation with an endoscope (1) which comprises a white light source (3, 16, 37), a fluorescence stimulator (4, 35) emitting a light bundle and an analyser (5, 15, 43), the white light source (3, 16, 37) comprises a device for the periodic intensity change of the light bundle exiting from the white light source (3, 16, 37), and the analyser (5, 15, 43) may be activated with the intensity of the light bundle of the white light source (3, 16, 37) approaching zero and may be deactivated with a visible light impulse, **characterised in that** during diagnosis a continuous light bundle is constantly emitted from the fluorescence stimulator (4, 35) onto the tissue.

2. An arrangement according to claim 1, **characterised in that** the white light source (3, 16, 37) is a constituent part of a stroboscope.

3. An arrangement according to claim 1 or 2, **characterised in that** the fluorescence stimulator (4) is a laser.

4. An arrangement according to claim 1 or 2, **characterised in that** the 9~5 fluorescence stimulator is an arc lamp.

5. An arrangement according to one of the claims 1 to 4, **characterised in that** a filter (19, 42) is allocated to the analyser (5, ~5, 43), which selectively blocks out the excitation wavelength exiting the fluorescence stimulator (4, 35).

## Revendications

1. Dispositif pour le diagnostic de tissus malins par observation de fluorescence à l'aide d'un endoscope (1), qui comporte une source de lumière blanche (3, 16, 37), un stimulateur de fluorescence (4, 35), qui délivre un faisceau de lumière, et un analyseur (5, 15, 43), que la source de lumière (3, 16, 37) comporte un dispositif pour modifier périodiquement l'intensité du faisceau de lumière sortant de la source de lumière blanche (3, 16, 37) et que l'analyseur (5, 15, 43) peut être activé lorsque l'intensité du faisceau de lumière de la source de lumière blanche (3, 16, 37) se rapproche de zéro, et peut être désactivé dans le cas d'une impulsion de lumière visible, **caractérisé en ce qu'**un faisceau de lumière continu est envoyé en permanence par le stimulateur de fluorescence (4, 35) sur le tissu, pendant le diagnostic.

2. Dispositif selon la revendication 1, **caractérisé en ce que** la source de lumière blanche (3, 16, 37) fait partie d'un stroboscope.

3. Dispositif selon la revendication 1 ou 2, **caractérisé en ce que** le stimulateur de fluorescence (4) est un laser.

4. Dispositif selon la revendication 1 ou 2, **caractérisé en ce que** le stimulateur de fluorescence est une lampe à arc.

5. Dispositif selon l'une des revendications 1 à 4, **caractérisé en ce qu'**à un analyseur (5, 15, 43) est associé un filtre (19, 42), qui occulte sélectivement la longueur d'onde d'excitation qui est délivrée par le stimulateur de fluorescence (4, 35).
